# EUROPEAN PATENT APPLICATION

(11) **EP 0 824 927 A2**
(43) Date of publication of application: **25.02.1998**
(21) Application number: 97830426.9
(22) Date of filing: 19.08.1997
(51) Int. Cl.: A61M 15/00

(54) **Smoke cessation aid**

(30) Priority: 19.08.1996 IT LU960012
(71) Applicant: Belli, Guido, 55042 Forte Dei Marmi, (Lucca) (IT)
(72) Inventor: Belli, Guido, 55042 Forte Dei Marmi, (Lucca) (IT)
(74) Representative: Bardini, Marco Luigi

(57) **Abstract**

A smoke cessation aid device comprising a part (3) shaped substantially as a cigarette holder having atomizing means inside, a reservoir (1) containing the substance (5) to be injected in the oral cavity and dispensing means (2) which put the part (3) into communication with the reservoir. The elimination of the dependence is obtained both thanks to the atomized and inhaled substances and to the device structure, the use of which reproduces the movements similar to those accompanying the act of smoking a cigarette.

## Description

The present invention relates to a smoke cessation aid device of the type which involves the dispensing of atomized substances in the oral cavity as a substitute for the smokes produced by the tobacco combustion in the oral cavity.

The problem of tabagism and of the research of solutions for assisting in the reduction of smoke dependency has been always at the centre of numerous studies and searches which approached to it substantially from a pharmaceutical standpoint and a physical standpoint. According to a pharmaceutical approach searches have focused in the development of molecules and pharmaceutical preparations to be administered orally on transdermally suitable of interacting with nicotine receptors and inducing a reaction of rejection to smoke and components thereof in the patient. According to a physical approach the proposed devices are based on the combined use of substantially inert aids or mechanical systems involving diffusion, filtration or inhalation associated to reservoirs containing one or more pharmacologically active substances or anyway capable of interacting with the organism in such a way to eliminate the smoke dependence. As far as the mechanical devices are concerned, various systems have been suggested designed to perform a protective action (cigarette inert substitutives), a limiting action (cigarette holders acting as active or passive filters) and a deterrent action (cigarette holders acting according to inhalation or activation mechanisms).

The cigarette holders having an active or passive filtering action aim to solve the problem of removal of noxious substances and combustion residues present in the cigarette smoke. A cigarette holder of this type is disclosed in FR-A-2598891 and comprises two cylinders connected to one another. The first cylinder acts as a support for the cigarette that is inserted in a special seat, while the second cylinder is the member acting as a filter. The cylinders communicate to each other through a number of aeration holes through which the smoke passes with the consequent dispersion, filtration and removal of the heavy particles present in the smoke. A further action of removal of the combustion residues and noxious substances present in the smoke is ensured by a filter arranged in the second cylinder. The device can retain the harmful substances present in the smoke, but cannot be used as deterrent or as a system for inducing the smoke habit cessation.

The cigarette holders acting as deterrents against the tabagism found their action on the combined use of inert supports and pharmaceutically or therapeutically active chemical substances. JP-A-02002331 discloses the use of a membrane permeable to a gas the passage of which through the filtering wall is controlled by the same membrane.

The inert support, i.e. the cigarette, can be made of paper of cellulose film; on the inside, a special reservoir contains a mixture of nicotine and tobacco extract which is taken by inhalation. The diffusion of the mixture of the substances present in the reservoir takes place by permeation of the gas through the membrane according to a passive diffusion mechanism.

A system similar to the previous one is disclosed in US-A-4807648 claiming the use of a solution containing nicotine and other flavouring substances mixed to a gummy preparation similar to a chewing-gum. The preparation is contained in a prosthesis in the shape of a cigarette. In this way the device can take the place of a cigarette partly ensuring to the user the same sensations and physiological/psychological satisfaction provided for by a cigarette while limiting the damages produced by the smoke.

DE-A-3506406 discloses a solution for getting a patient out of the habit of smoking, of drinking and of an excessive feeding substantially consisting in the use of a capsule with a controlled release valve which is introduced in a cannule or a flexible support. The operation of the device is controlled by the aspiration through a valve which, therefore, can perform the function of cigarette holder or suction tube for drinks or liquid foods, according to the particular use and shape. Inside the cannula or cigarette holder there is housed the capsule containing flavouring substances, essences, flower extracts, caffeine, nicotine extracts, vitamines drugs. The capsule valve opens due to the suction and the flavours and other substances present therein are released.

WO91/18636 discloses the use of an impermeable capsule containing nicotine to be housed in a cigarette holder having a inner part with a sharp edge for piercing the capsule when applied to the holder. The nicotine vapour are inhaled by aspiration in this case too.

None of the above described system or other similar smoke cessation aids are satisfactory both from the physical and psychological point of view at the same time, because, even if the smoker can feel some degree of satisfaction due to the action of the inhaled or, anyway, ingested substance on the organism, he is, however, insufficiently satisfied from the psychological point of view due to the structural and functional differences of the known devices with respect to a common cigarette.

The object of the present invention is to provide a device for assisting in smoke cessation of the type which involves the dispensing of at least an active substance in the oral cavity of the user, whose use is such as to provide the user with a complete psychological satisfaction as if would really keep a cigarette in one's mouth.

This object is attained with the smoke cessation aid according to the present invention whose main features are set forth in the appended claim 1.

Further characteristics and advantages of the smoke cessation aiding device according to the present invention will become apparent from the following description of an exemplifying and not limiting embodiment thereof, made with reference to the attached drawing in which:
- Figure 1 is a side view of the device of the invention;
- Figure 2 is a partial longitudinal section of the device of figure 1;
- Figure 3 is an exploded view of the device of figure 1;
- Figure 4 is a variation of a particular of the device according to the invention.

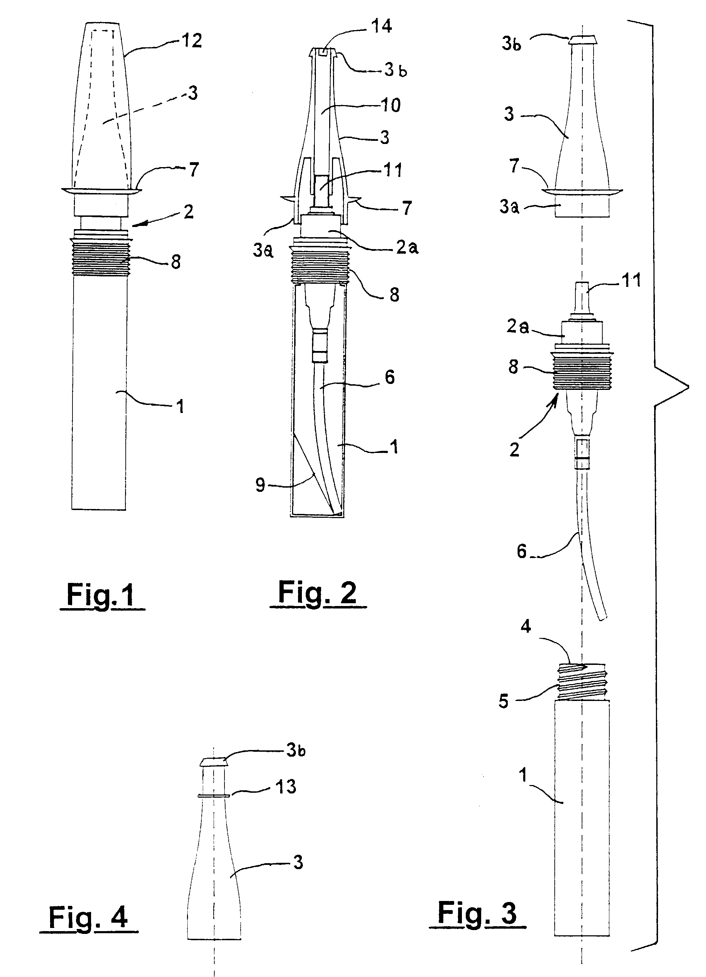

With reference to the above mentioned figures, 1 indicates a reservoir for a substance or a mixture of substance to be dispensed, 2 indicates a dispensing device for said substances applied to an opening of reservoir 1, and 3 a mouthpiece having an external shape substantially equal to that of a conventional cigarette holder and communicating with the inside of reservoir through dispensing device 2. Preferably reservoir 1 has an elongated, cylindrical shape so as to look like a cigarette as to the form and dimensions. Reservoir 1 has an opening 4 in correspondence to a base from which there extends a threaded portion 5 for engaging with a corresponding threaded seat, not shown, of dispensing device 2 to allow the connection of the latter to reservoir 1. In a possible embodiment, dispensing device 2 consists of a micropump of the commercial type (for example, the micropump produced by ELETTROPLASTICA S.r.I., Rozzano-Milano, Italy) which is not described in detail, as of a well known type. A small tube 6 extends from dispensing device 2 within reservoir 1 to draw off from the bottom thereof. Advantageously the bottom of reservoir 1 has a conical shape at the inner side or, as shown in figure 2, comprises a wall 9 inclined with respect to the longitudinal axis of reservoir 1, so that the liquid contained therein can be drawn off even when the reservoir is nearly empty.

As an alternative, dispensing device 2 can be constituted by a conventional spray system comprising a propellant gas in the mixture of substances contained in reservoir 1.

Mouthpiece 3 is connected to body 2a of dispensing device 2 through an end 3a close to which a perimetrical fin 7 is provided. Fin 7 extends radially from the external surface of mouthpiece 3. Dispensing device 2 has a corrugated, optionally recessed, surface 8 in correspondence to at least a portion of its external surface, to make easier the use of the device, as explained hereunder. Obviously, the corrugation can be provided, as an alternative on the wall of reservoir in an intermediate position.

The other end 3b of mouthpiece 3 is enlarged, as in a conventional cigarette holder to make easier the engagement between the lips, while in mouthpiece 3 there is formed a longitudinal conduit 10 extending from end 3a to end 3b, in which a dispensing device nozzle 11, also acting as axially and elastically sliding control pushbutton, is forcibly engaged. Within conduit 10, in particular in correspondence to enlarged end 3b, atomizing means, for example of the diaphragm type, schematically shown at 14, are provided. These atomizing means are not described in details as of the type commonly used in atomizing devices for pharmaceutical use. By axially sliding mouthpiece 3, dispensing device 2 is actuated and the liquid drawn off from reservoir 1 is dispensed through conduit 10 in the oral cavity under atomized form.

An airtight cap 12 is finally provided to protect the mouthpiece part intended for being placed in the user's mouth, when not in use.

In the use, the user catches the device according to the invention between the forefinger and the middle finger in correspondence to the corrugated surface 8 and with the forefinger in correspondence to the possible recess formed thereon. The thumb is then leant against fin 7 to press it and cause the actuation of dispensing device 2, thereby the atomized flow of substances contained in reservoir 1 is passed in the user mouth through conduit 10. In this way, the smoker, while doing a typical gesture of placing a cigarette in one's mouth, press fin 7 thus causing mouthpiece 3 to slide with respect to reservoir 1 and dispensing the substances contained in reservoir 1 into one's mouth. The smoker, therefore, has the feeling that he/she is actually smoking, which turns out to be psychologically gratifying.

According to a variation of the invention, the operation of the dispensing device can be occur also by axial compression of the mouthpiece against the user lips. As shown in figure 4, in place of perimetrical fin 7, mouthpiece 3 is provided with an annular rib 13 radially extending from its surface at an intermediate point, in particular near enlarged end 3b. In this way, while keeping the device between the forefinger and the middle in correspondence to corrugated surface 8, as in the previous case, and pushing it axially toward the mouth, rib 3 abuts against the smoker lips, thereby causing the sliding of mouthpiece 3 with respect to reservoir 1 and the operation of dispensing device 2.

In both cases, the administration of the atomized substances does not requires an aspiration by the smoker. However, since aspiration is a typical action made by the smoker, the mouthpiece of the device according to the invention will be provided with air outlet holes, not shown, in correspondence to the enlarged end 3a and air intake holes, not shown in correspondence to the side surface of the mouthpiece. In this way, even if this expedient is useless from the functional standpoint, the smoker can breath in through the mouthpiece with considerable psychological advantage.

The device according to the invention can be used for dispensing atomized substances either active principles or inert substances, either in independent form or in combination thereof, capable of being absorbed by the mouth mucosae and/or the respiratory system. The selection of the substances to be used with the device of the invention is a function of the applications and desired effects on the organism. Many compositions, extracts, homeopathic drugs, mixtures for inducing the elimination of the habit to smoke and other drugs have been described and can be used with the device according to the present invention.

The device according to the invention can be made available in a disposable form, i.e. ready to use with the substances to be dispensed already placed in reservoir 1. As an alternative, the device of the invention can be made available in a partially disposable form, in the sense that only the reservoir containing the substances to be dispensed is each time replaced, whilst the mouthpiece and the dispensing device can be used a number of times. According to a further variation, the substance to be dispensed can be made available in ampoules or similar containers, by which reservoir 1 can be filled once its content runs out.

The original feature of the device according to the invention consists in that it allows for a simultaneous satisfaction of the various needs of the smoker, wishing to stop smoking, in a pleasant and not traumatic way. In fact, it allows the primary inconvenience of the abstinence to be overcome in various ways both by the administration of gradually reduced doses of drugs, and by the administration of substitute substances, in both cases optionally added with other medicinal substances or flavouring substances (such as, tobacco essence), suitable for satisfying the gustative needs of the user. Furthermore the consistency and density of the atomized dose dispensed each time in the oral cavity has the feature of a greater similarity to smoking a cigarette than the gaseous substances delivered by the known devices.

The dispensing of the substances, both active and inert, occurs according to an easy and automatic mechanism reminding the act of smoking and just for this reason the device is useful in particular in assisting or inducing the elimination of the habit to drugs taken by inhalation such as tobacco. However the device can be used to eliminate the adaptation to other drugs taken orally or through other ways, in order to modify abnormal physiological conditions or life styles negatively affecting the organism. It is, therefore, useful to administer substances effective in the treatment of depression, bulimia, addiction upon light and heavy drugs, alcoholism.

It will be understood for a person skilled in the art that many variation can be made to the device according to the invention without departing from the scope of the invention. For example, the mouthpiece, the dispensing device and the reservoir can be connected to one another in different ways from that shown hereinbefore, for example they can be connected with fixed joints or snap fitted or they can be made in a single piece and in any case they will have to be connected in such a way as to avoid any losses of the substances contained in the device. Finally, even if in the present description reference has been made mainly o the use of the device of the invention as a smoke cessation aid, it is clear that it can be used also to assist in eliminating the habit to other drugs, in particular when the dependence is associated to the smoke of a cigarette, or to administer active principle effective in the treatment of diseases related to the use of drugs, in association to substances directed to eliminate the habit to these drugs.

## Claims

1. A smoke cessation aid device, characterized in that it comprises a reservoir (1) for at least a substance capable of assisting in eliminating the habit to smoke, dispensing means (2) of said substance arranged at an opening (4) formed in said reservoir (1), a tubular member (3), substantially in the shape of a cigarette holder, comprising atomizing means, and communicating with said reservoir through said dispensing means (2) and connected to the latter in such a way to cause the operation of said dispensing means, and therefore the introduction of said substance in an atomized form within the oral cavity, as a consequence of a relative sliding movement of said tubular member with respect to said reservoir, said movement been manually controlled.

2. The smoke cessation aid device according to claim 1, in which said reservoir (1) has a cylindrical shape substantially similar to that of a cigarette, said opening (4) being formed in correspondence to a base thereof, said tubular member (3) being substantially coaxial to said reservoir.

3. The device according to the previous claims, in which said dispensing means (2) comprise a micropump.

4. The device according to claims 1 and 2, in which said dispensing means (2) are of the spray type.

5. The device according to the previous claims, in which said atomizing tubular member has a perimetrical fin (7) extending along to one end thereof (3a) connected to said dispensing means (2), on said fin the user pressing with a finger to cause said relative sliding movement.

6. The device according to the claims 1 to 4, in which said atomizing tubular member has an annular rib (13) close to the end intended to be put in the user's mouth, said rib abutting against the user's lips during said relative sliding movement.

7. The device according to the previous claims, in which a corrugated surface (8) is provided on said reservoir or said dispensing means to allow to catch the device between two fingers.

8. The device according to claim 7, in which at least a recess is provided on said corrugated surface (8).

9. The device according to the previous claims, in which the bottom of said reservoir has a conical shape or is inclined to allow the contained substance to be drawn off even if the reservoir is nearly empty.

10. The device according to the previous claims, in which said dispensing means comprise a body (2a) and a nozzle (11) acting also as a operating push-button, sliding elastically and axially with respect to said body (2a) and suitable for forcibly engaging within a passing through conduit (10) of said tubular member.
